# EUROPEAN PATENT SPECIFICATION

(11) **EP 4 248 928 B1**
(45) Date of publication and mention of the grant of the patent: **03.12.2025**
(21) Application number: 21915100.8
(22) Date of filing: 15.12.2021
(51) Int. Cl.: A61F 13/53, C08F 8/12

(54) **COMPOSITE ABSORBENT AND SANITARY MATERIAL**
ZUSAMMENGESETZTES ABSORBIERENDES UND SANITÄRES MATERIAL
ABSORBANT COMPOSITE ET MATÉRIAU HYGIÉNIQUE

(30) Priority: 29.12.2020 JP 2020219812
(43) Date of publication of application: 27.09.2023
(73) Proprietor: Unicharm Corporation, Shikokuchuo-shi, Ehime 799-0111 (JP)
(72) Inventor: KIKUCHI, Kyo, Kanonji-shi, Kagawa 769-1602 (JP); KINOSHITA, Akie, Kanonji-shi, Kagawa 769-1602 (JP); NAKASHITA, Masashi, Kanonji-shi, Kagawa 769-1602 (JP); GODA, Hiroki, Kanonji-shi, Kagawa 769-1602 (JP); UKEGAWA, Kazuo, Kanonji-shi, Kagawa 769-1602 (JP); KUNITOMO, Teruo, Kanonji-shi, Kagawa 769-1602 (JP)
(74) Representative: Dolleymores
(86) International application number: PCT/JP2021/046323
(87) International publication number: WO 2022/145239

(56) References cited:
- EP-A1- 0 993 310
- EP-B1- 0 993 310
- JP-A- 2002 505 702
- JP-A- 2009 007 550
- JP-A- 2017 164 356
- JP-A- 2017 164 356
- US-A1- 2020 383 846

## Description

### FIELD

The present invention relates to a composite absorbent body and a sanitary product having the same.

### BACKGROUND

As sanitary products such as disposable diapers and sanitary napkins, there are known articles containing a porous material such as a sponge material or a superabsorbent polymer (so-called "SAP") having a high absorption amount as an absorbent body.

For example, Patent Literature 1 discloses an absorbent article containing a polymeric foam material (porous material) having a hydrophilic flexible structure of interconnected open cells, and Patent Literature 2 discloses an absorbent article using an absorbent body formed by combining absorbent resin particles (superabsorbent polymer) having an excellent absorption amount and hydrophilic fibers such as pulp fibers having an excellent absorption rate. US 2020/383846 A1 and JP2017164356 A disclose absorbent articles.

### [PATENT LITERATURE]

[Patent Literature 1] Japanese Patent No. 3231320
[Patent Literature 2] PTC International Publication WO 2013/018571

### SUMMARY

### [TECHNICAL PROBLEM]

The absorbent material used in such a conventional absorbent body has the following features. For example, a porous material such as that disclosed in Patent Literature 1 absorbs water by taking the water into a plurality of pores having predetermined spaces. Accordingly, although the water can be instantaneously absorbed by utilizing a capillary phenomenon, the spaces capable of absorbing the water are limited to the predetermined spaces formed by the plurality of pores, and thus it is difficult to absorb a large amount of water.

On the other hand, since the superabsorbent polymer (SAP) as disclosed in Patent Literature 2 takes in water while widening (that is, expanding) the space corresponding to the volume of the water taken in, a large amount of water can be retained (that is, the water retention capacity is high). However, the water absorption rate is low, and thus it is difficult to instantaneously absorb the water.

Further, in order to compensate for the disadvantages of these absorbent materials, it is conceivable that these absorbent materials are used at the same time. However, for example, in a case where a large amount of bodily fluid such as urine or menstrual blood is discharged from a wearer of a sanitary product, the absorption capacity (absorption limit) of the porous material may be exceeded in the initial stage of absorption. In addition, there is a risk that high absorption performance cannot be obtained as a result of the fact that the bodily fluid that has not been absorbed by the porous material is not immediately absorbed even in the SAP.

The present invention was achieved in view of such problems, and an aspect of the present invention is to provide an absorbent body which has high absorption performance and is capable of instantaneously absorbing a large amount of bodily fluid.

### [SOLUTION TO PROBLEM]

One aspect (Aspect 1) of the present invention is defined in claim 1.

In the composite absorbent body according to Aspect 1, when a polymer absorbent having a hydrophilic continuous skeleton and continuous pores absorbs a bodily fluid such as urine or menstrual blood, the hydrophilic continuous skeleton instantaneously takes in the bodily fluid by osmotic pressure and expands. Due to this, the volume of the continuous pores can be enlarged, and further the bodily fluid can be taken into the expanded continuous pores. Therefore, a large amount of bodily fluid can be instantaneously absorbed, and further the absorbed bodily fluid can be transferred to a superabsorbent polymer (SAP) having a high water retention capacity and can be steadily held in the SAP.

Accordingly, the composite absorbent body of the present aspect can exhibit high absorption performance as an absorbent body.

Further, another aspect (Aspect 2) of the present invention is:
the composite absorbent body according to Aspect 1, wherein
a volume of the expanded polymer absorbent is larger than a volume of the water taken in at the time of water absorption of the polymer absorbent at a water retention limit.

In the composite absorbent body of the present aspect, since the polymer absorbent can take a larger amount of water into the continuous pores, the composite absorbent body can exhibit higher absorption performance as an absorbent body.

Still another aspect (Aspect 3) of the present invention is:
the composite absorbent body according to Aspect 1 or 2, wherein
a water absorption amount of the continuous pores is larger than a water absorption amount of the continuous skeleton in the polymer absorbent.

In the composite absorbent body of the present aspect, since the polymer absorbent can take a larger amount of water into the continuous pores, the composite absorbent body can exhibit higher absorption performance as an absorbent body.

Still another aspect (Aspect 4) of the present invention is:
the composite absorbent body according to any one of Aspects 1 to 3, wherein
a mass ratio between a water absorption amount of the continuous pores and a water absorption amount of the continuous skeleton in the polymer absorbent is 60:40 to 95:5.

The composite absorbent body of the present aspect enables water to be more steadily taken into the continuous skeleton and then taken into the continuous pores when the polymer absorbent absorbs the water, since the mass ratio between the water absorption amount of the continuous pores and the water absorption amount of the continuous skeleton in the polymer absorbent is within the above-described specific range.

Still another aspect (Aspect 5) of the present invention is:
the composite absorbent body according to any one of Aspects 1 to 4, wherein
the polymer absorbent has a relatively high absorption rate from immediately after start of water absorption until reaching a water retention limit, and a relatively low absorption rate after the water retention limit.

In the composite absorbent body of the present aspect, while in the initial stage of water absorption from immediately after the start of water absorption until reaching the water retention limit, the absorption rate of the polymer absorbent is relatively high and the bodily fluid can be instantaneously taken into the continuous skeleton, in the latter stage of water absorption after the water retention limit, the absorption rate of the polymer absorbent is relatively low and the bodily fluid can be steadily taken into the continuous pores expanded due to the expansion of the continuous skeleton. Therefore, the composite absorbent body can more reliably exhibit high absorption performance as an absorbent body.

Still another aspect (Aspect 6) of the present invention is:
the composite absorbent body according to any one of Aspects 1 to 5, wherein
the polymer absorbent is a monolithic absorbent.

In the composite absorbent body of the present aspect, since the polymer absorbent is a monolithic absorbent, the bodily fluid can be rapidly absorbed and the bodily fluid temporarily held in the polymer absorbent can be more steadily transferred to the SAP.

Still another aspect (Aspect 7) of the present invention is:
the composite absorbent body according to any one of Aspects 1 to 6, wherein
the polymer absorbent is a hydrolysate of a crosslinked polymer of a (meth)acrylic acid ester and a compound containing two or more vinyl groups in one molecule, and contains at least one or more -COONa groups.

In the composite absorbent body of the present aspect, since the polymer absorbent has the above-described specific configuration, the hydrophilic continuous skeleton easily extends (that is, easily expands), and the continuous pores are also easily widened when the bodily fluid is absorbed. Therefore, a larger amount of bodily fluid can be more rapidly taken into the continuous pores, and the composite absorbent body can more reliably exhibit further excellent absorption performance as an absorbent body.

Still another (Aspect 8) of the present invention is:
a sanitary product comprising:
the composite absorbent body according to any one of Aspects 1 to 7.

Since the sanitary product of the present aspect includes the composite absorbent body of any one of Aspects 1 to 7, excellent absorption performance as a sanitary product can be exhibited.

### [ADVANTAGEOUS EFFECT OF THE INVENTION]

According to the present invention, an absorbent body which has high absorption performance and is capable of instantaneously absorbing a large amount of bodily fluid can be provided.

### BRIEF DESCRIPTION OF DRAWINGS

FIG. 1 is a schematic plan view of a light incontinence pad 1 in an unfolded state when viewed in the thickness direction from the skin facing surface side.
FIG. 2 is a diagram illustrating a manufacturing process of an absorbent A which is an example of a polymer absorbent.
FIG. 3 is an SEM photograph of the absorbent A at a magnification of 50 times.
FIG. 4 is an SEM photograph of the absorbent A at a magnification of 100 times. FIG. 5 is an SEM photograph of the absorbent A at a magnification of 500 times.
FIG. 6 is an SEM photograph of the absorbent A at a magnification of 1000 times. FIG. 7 is an SEM photograph of the absorbent A at a magnification of 1500 times.
FIG. 8 is a cross-sectional photograph taken with a scanning electron microscope showing the appearance of the absorbent A before and after water absorption.
FIG. 9 is a CT image showing the absorption state of the absorbent A in each dropping amount of distilled water.
FIG. 10 is a CT image showing the absorption states of two types of SAPs in each dropping amount of distilled water.
FIG. 11 is a CT image showing the absorption state of an Infinity particle body in each dropping amount of distilled water.
FIG. 12 is a graph showing a relationship between the water absorption ratio and the volume change amount of the absorbent A which is an example of a polymer absorbent.

### DESCRIPTION OF EMBODIMENTS

Hereinafter, a preferable embodiment of a composite absorbent body of the present invention will be described in detail using a light incontinence pad 1 which is an example of a sanitary product to which the composite absorbent body is applied.

It should be noted that in the present specification, unless otherwise specified, an "object (for example, a light incontinence pad, a composite absorbent body, or the like) placed on a horizontal plane in an unfolded state is viewed in a thickness direction of the object from the upper side in the vertical direction (in a case where the object is a sanitary product, the top sheet side)" will be simply referred to as a "plan view".

It should be noted that in the present specification, the "lengthwise direction" refers to a "direction in which the length of a longitudinally elongated object (for example, a light incontinence pad, a composite absorbent body, or the like in an unfolded state) in a plan view is long", the "width direction" refers to a "direction in which the length of a longitudinally elongated object in a plan view is short", the "thickness direction" refers to a "direction vertical to the object placed on a horizontal plane in an unfolded state", and the lengthwise direction, the width direction, and the thickness direction have a relationship in which the respective directions are orthogonal to each other.

Further, in the present specification, unless otherwise specified, in the thickness direction of the light incontinence pad 1, a "relatively proximal side with respect to the skin surface of the wearer while the light incontinence pad 1 is put on" will be referred to as a "skin facing surface side," and a "relatively distal side with respect to the skin surface of the wearer while the light incontinence pad 1 is put on" will be referred to as a "non-skin facing surface side."

### [Light Incontinence Pad]

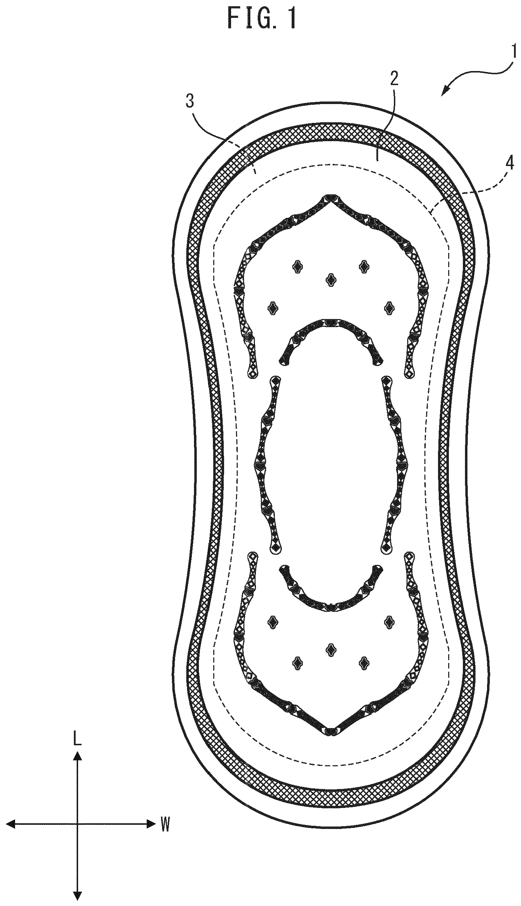

FIG. 1 is a schematic plan view of a light incontinence pad 1 in an unfolded state to which a composite absorbent body 4 according to an embodiment of the present invention is applied.

As shown in FIG. 1, in a plan view, the light incontinence pad 1 has a lengthwise direction L and a width direction W, and has a longitudinally elongated outer shape in which two lengthwise end edges protrude toward the outer side in the lengthwise direction in an arc shape. It should be noted that the outer shape of the light incontinence pad 1 is not limited to such an embodiment, and, as long as the light incontinence pad 1 has a longitudinally elongated shape, any shape (for example, an elliptical shape, a rectangular shape, a hourglass shape, a gourd shape, and the like) can be employed according to various uses, usage modes, and the like.

The light incontinence pad 1 basically includes, in a thickness direction, a liquid-permeable top sheet 2 that forms a surface on a skin facing surface side of the light incontinence pad 1, a back sheet 3 that forms a surface on a non-skin facing surface side of the light incontinence pad 1, and a composite absorbent body 4 that is positioned between the top sheet and the back sheet. Further, the light incontinence pad 1 further includes an adhesive portion (not shown) that is arranged on the surface of the non-skin facing surface side of the back sheet 3 and adhesively fixes the light incontinence pad 1 to the inner surface of clothing such underwear of a wearer.

It should be noted that the configuration of the light incontinence pad 1 is not limited to the above-described configuration, and, for example, the light incontinence pad 1 may include a pair of side sheets for forming leakproof walls, which are positioned at both end portions of the light incontinence pad 1 in the width direction W and arranged to extend in the lengthwise direction L, at a position on the skin facing surface side with respect to the top sheet 2, and a plurality of elastic members which are arranged along the lengthwise direction L in each of the pair of side sheets.

Further, in the light incontinence pad 1, the composite absorbent body 4 is formed of a water-absorbent member that is positioned between the top sheet 2 and the back sheet 3 and is capable of absorbing a bodily fluid such as urine or menstrual blood discharged from the wearer and transmitted through the top sheet 2, and the composite absorbent body 4 includes a polymer absorbent having a hydrophilic continuous skeleton and continuous pores, and a superabsorbent polymer (SAP).

Further, the polymer absorbent exhibits a unique water absorption behavior that, when water is absorbed, the water is taken into the continuous skeleton and then taken into the continuous pores.

In the composite absorbent body 4, when a polymer absorbent having a hydrophilic continuous skeleton and continuous pores described above absorbs a bodily fluid such as urine or menstrual blood, the hydrophilic continuous skeleton instantaneously takes in the bodily fluid by osmotic pressure and expands. Due to this, the volume of the continuous pores can be enlarged, and further the bodily fluid can be taken into the expanded continuous pores. Therefore, a large amount of bodily fluid can be instantaneously absorbed, and further the absorbed bodily fluid can be transferred to SAP having a high water retention capacity and can be steadily held in the SAP.

Accordingly, the composite absorbent body 4 can exhibit high absorption performance as an absorbent body.

Therefore, the light incontinence pad 1 including such a composite absorbent body 4 can also exhibit excellent absorption performance as a light incontinence pad.

Hereinafter, various configurating members of a sanitary product to which the composite absorbent body of the present invention is applied will be described in more detail using the above-described light incontinence pad 1.

### [Top sheet]

In the above-described light incontinence pad 1, the top sheet 2 has a longitudinally elongated outer shape that extends from one side end edge to the other side end edge in the lengthwise direction L of the light incontinence pad 1, and extends over the vicinity of the one side end edge to the vicinity of the other side end edge in the width direction W of the light incontinence pad 1 in a plan view as shown in FIG. 1. The top sheet 2 is formed of a liquid-permeable sheet-like member arranged at a position on the skin facing surface side in the thickness direction of the light incontinence pad 1 and forms a contact surface capable of coming into contact with the skin of the wearer, that is, the surface on the skin facing surface side of the light incontinence pad 1.

Further, as shown in FIG. 1, the top sheet 2 has a slightly larger size in the lengthwise direction L and the width direction W compared with the composite absorbent body 4 arranged on the non-skin facing surface side of the top sheet 2, and is joined to the back sheet 3 positioned on the non-skin facing surface side in the peripheral edge portion.

In the present invention, the outer shape, various dimensions, basis weight, and the like of the top sheet are not particularly limited, as long as they can be used for the top sheet of a sanitary product, and any shape, various dimensions, basis weight, and the like according to the desired liquid permeability, texture, flexibility, strength, and the like can be employed.

### [Back sheet]

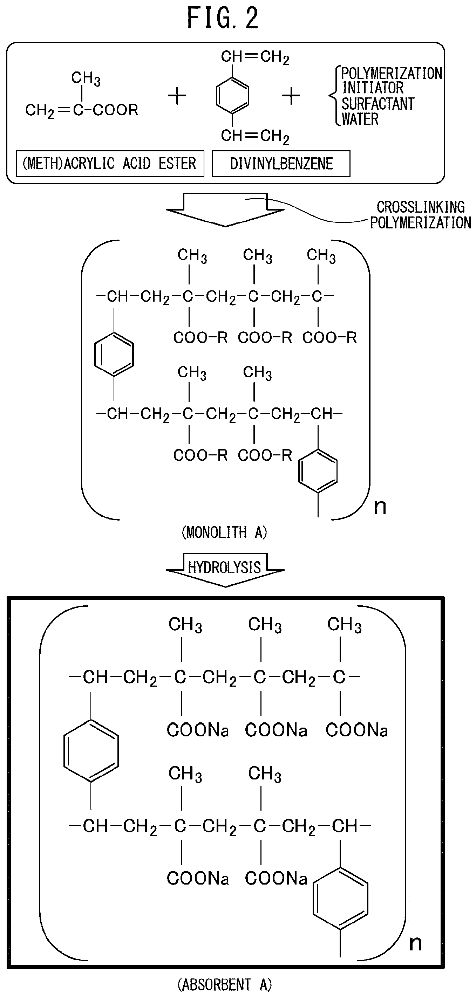

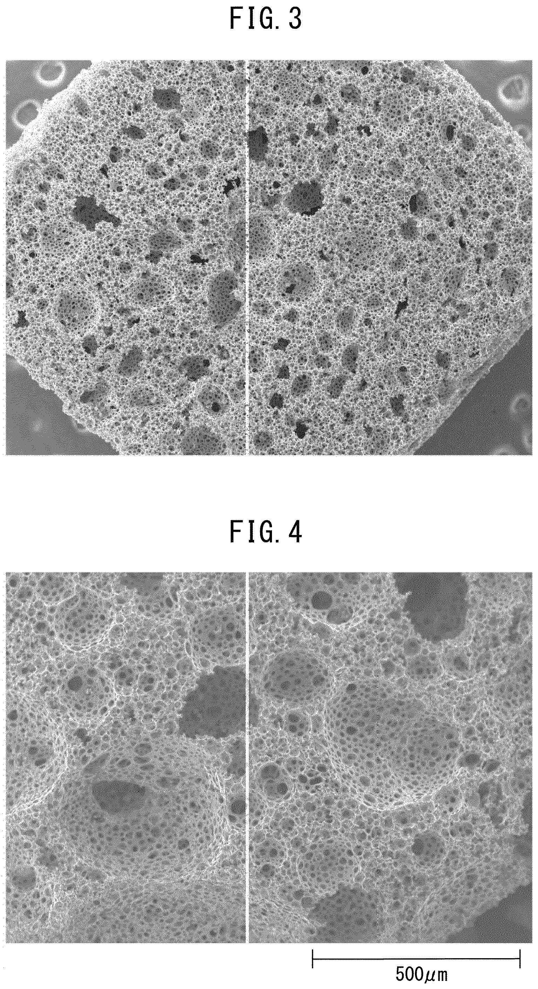

In the above-described light incontinence pad 1, the back sheet 3 has a longitudinally elongated outer shape that extends from one side end edge to the other side end edge in the lengthwise direction L of the light incontinence pad 1 and extends from the one side end edge to the other side end edge in the width direction W of the light incontinence pad 1 in a plan view as shown in FIG. 2. As shown in FIG. 3, the back sheet 3 is arranged at a position on the non-skin facing surface side in the thickness direction of the light incontinence pad 1, and is formed of a liquid-impermeable sheet-like member that forms the non-skin facing surface of the light incontinence pad 1 and prevents a bodily fluid such as urine or menstrual blood that has permeated the composite absorbent body 4 from leaking out from the light incontinence pad 1.

In the present invention, the outer shape, various dimensions, basis weight, and the like of the back sheet are not particularly limited, as long as they can be used for the back sheet of the sanitary product, and any shape, various dimensions, basis weight, and the like according to the desired leakage preventing performance, breathability, strength, and the like can be employed.

### (Composite Absorbent Body)

As shown in FIG. 1, in a plan view, in the above-described light incontinence pad 1, the composite absorbent body 4 has a longitudinally elongated outer shape that extends in a wide region in the lengthwise direction L from the vicinity of one side end edge to the vicinity of the other side end edge in the lengthwise direction L, centering on the central part of the light incontinence pad 1 in the lengthwise direction L and the width direction W, and also extends in the width direction W in a wide region from the vicinity of the one side end edge to the vicinity of the other side end edge in the width direction W, and in which two lengthwise end edges protrude in an arc shape toward the lengthwise outer side in a plan view, as shown in FIG. 1.

More specifically, the composite absorbent body 4 has a constricted portion having a relatively short widthwise length compared with other portions in the lengthwise central part in a plan view, and further has a minimum width portion having the minimum width of the composite absorbent body 4 in the constricted portion, and a maximum width portion having the maximum width of the composite absorbent body 4 on the lengthwise outer side of the constricted portion.

The composite absorbent body 4 is formed of a predetermined water-absorbent member that is arranged between the top sheet 2 and the back sheet 3 in the thickness direction of the light incontinence pad 1 and is capable of absorbing and holding a bodily fluid such as urine or menstrual blood that has permeated the top sheet 2 and is formed of a water-absorbent material such as a polymer absorbent, hydrophilic fibers, or superabsorbent polymer, which will be described later, and a sheet such as a tissue that holds the water-absorbent material. That is, the composite absorbent body means a water absorbent member formed of a water-absorbent material that can absorb and hold a bodily fluid, and a sheet that holds the water-absorbent material.

It should be noted that in the light incontinence pad 1, the composite absorbent body 4 is joined to each of the top sheet 2 and the back sheet 3 with any adhesive such as a hot-melt adhesive.

Further, the composite absorbent body 4 includes the polymer absorbent having a hydrophilic continuous skeleton and continuous pores and having a unique water absorption behavior, and a superabsorbent polymer as described above. The polymer absorbent will be described later, but the superabsorbent polymer is a powder or granular matter formed of a superabsorbent polymer such as a sodium acrylate copolymer well-known in the art, and is referred to as super absorbent polymer (SAP).

It should be noted that the composite absorbent body 4 may include only the polymer absorbent and the superabsorbent polymer as the water-absorbent material, or may further include a water-absorbent material well-known in the art in addition to these materials. Examples of such a water-absorbent material include hydrophilic fibers and the like, and more specifically, cellulose-based fibers such as pulp fibers (e.g., pulverized pulp), cotton, rayon, and acetate and the like can be given.

It should be noted that the composite absorbent body 4 may have a configuration in which such a polymer absorbent, a superabsorbent polymer, and any water-absorbent material are covered with a wrap sheet such as a tissue having hydrophilicity.

It should be noted that, in the present invention, the outer shape, various dimensions, basis weight, and the like of the composite absorbent body are not particularly limited as long as the effects of the present invention are not impaired, and any outer shape, various dimensions, basis weight, and the like according to the desired water absorption, flexibility, strength, and the like can be adopted.

Hereinafter, the polymer absorbent used in the composite absorbent body of the present invention will be described in more detail.

### [Polymer Absorbent]

In the present invention, the polymer absorbent is not particularly limited as long as the absorbent has a hydrophilic continuous skeleton and continuous pores, and exhibits a unique water absorption behavior that, when water is absorbed, the water is taken into the continuous skeleton and then taken into the continuous pores. As such a polymer absorbent, for example, there is provided a polymer compound that is a hydrolysate of a crosslinked polymer of two or more monomers containing at least a (meth)acrylic acid ester and has at least one or more hydrophilic groups in the functional group. More specifically, there is provided a polymer compound that is a hydrolysate of a crosslinked polymer of a (meth)acrylic acid ester and a compound containing two or more vinyl groups in one molecule and has at least -COONa group. Such a polymer absorbent is an organic porous body having at least one or more -COONa groups in one molecule and may further have a -COOH group. In the skeleton of the porous body, -COONa groups are substantially uniformly distributed.

When the polymer absorbent is a polymer compound that is a hydrolysate of a crosslinked polymer of such (meth)acrylic acid ester and a compound containing two or more vinyl groups in one molecule and contains at least one or more a -COONa groups, as described later, the hydrophilic continuous skeleton easily extends (that is, easily expands) when a bodily fluid such as urine or menstrual blood is absorbed, and the continuous pores are easily widened. Therefore, a larger amount of bodily fluid can be more rapidly taken into the continuous pores, and further excellent absorption performance can be exhibited as an absorbent body.

It should be noted that in the present specification, the term a (meth)acrylic acid ester refers to an acrylic acid ester or a methacrylic acid ester.

In the polymer absorbent formed of such a hydrolysate of a crosslinked polymer of a (meth)acrylic acid ester and divinylbenzene, a hydrophilic continuous skeleton is formed of an organic polymer having at least a -COONa group, and has continuous holes (continuous pores) that serve as absorption sites for a liquid to be absorbed (that is, a bodily fluid such as urine or menstrual blood) are provided between the skeletons.

It should be noted that since in the hydrolysis treatment, the -COOR group (that is, the carboxylic acid ester group) of the crosslinked polymer is converted into a the -COONa group or the -COOH group (refer to FIG. 2), the polymer absorbent may have the -COOR group.

The presence of the -COOH group and the -COONa group in the organic polymer that forms the hydrophilic continuous skeleton can be confirmed by analysis through infrared spectrophotometry or a method of quantifying a weakly acidic ion exchange group.

Here, FIG. 2 is a diagram illustrating a manufacturing process of an absorbent A which is an example of a polymer absorbent. In FIG. 2, the upper figure shows polymer constituent raw materials, the middle figure shows a monolith A as a crosslinked polymer of a (meth)acrylic acid ester and divinylbenzene, and the lower figure shows the absorbent A obtained by subjecting the monolith A in the middle figure to hydrolysis and drying.

Hereinafter, an absorbent A formed of a hydrolysate of a crosslinked polymer of a (meth)acrylic acid ester and divinylbenzene, which is an example of a polymer absorbent, will be described.

It should be noted that the polymer absorbent is not limited to such an absorbent A, and may be a hydrolysate of a crosslinked polymer of a (meth)acrylic acid ester and a compound having two or more vinyl groups in one molecule, or a hydrolysate of a crosslinked polymer of two or more types of monomers containing at least (meth)acrylic acid ester, or the like.

However, when the polymer absorbent is a monolithic absorbent, there are advantages that the bodily fluid can be rapidly absorbed and the bodily fluid temporarily held in the polymer absorbent can be more steadily transferred to the SAP.

It should be noted that in the following description, the "monolith A" is an organic porous body formed of a crosslinked polymer of a (meth)acrylic acid ester and divinylbenzene before being subjected to hydrolysis, and will also be referred to as a "monolithic organic porous body".

The "absorbent A" is a hydrolysate of a crosslinked polymer (monolith A) of a (meth)acrylic acid ester and divinylbenzene after being subjected to hydrolysis and drying. It should be noted that the absorbent A is assumed to be in the dry state in the following description.

First, the structure of the absorbent A will be described.

As described above, the absorbent A has a hydrophilic continuous skeleton and continuous pores. The absorbent A, which is an organic polymer having a hydrophilic continuous skeleton, is obtained by performing crosslinking polymerization using (meth)acrylic acid ester, which is a polymerization monomer, and divinylbenzene, which is a crosslinking monomer, and then hydrolyzing the obtained crosslinked polymer (monolith A) as shown in FIG. 2.

The organic polymer that forms the hydrophilic continuous skeleton has, as constituent units, polymerization residue of an ethylene group (hereinafter, referred to as a "constituent unit X") and a crosslinked polymerization residue of divinylbenzene (hereinafter, referred to as a "constituent unit Y").

Further, the polymerization residue (constituent unit X) of the ethylene group in the organic polymer that forms the hydrophilic continuous skeleton has a -COONa group or both of the - COOH group and the -COONa group generated by the hydrolysis of the carboxylic acid ester group. It should be noted that, in a case where the polymerization monomer is a (meth)acrylic acid ester, the polymerization residue of the ethylene group (constituent unit X) has a -COONa group, a -COOH group, and an ester group.

In the absorbent A, the ratio of the crosslinked polymerization residue (constituent unit Y) of divinylbenzene in the organic polymer that forms the hydrophilic continuous skeleton is, for example, 0.1 to 30 mol %, and preferably 0.1 to 20 mol %, with respect to all of the constituent units. For example, in the absorbent A containing butyl methacrylate as the polymerization monomer and divinylbenzene as the crosslinking monomer, the ratio of the crosslinked polymerization residue (constituent unit Y) of divinylbenzene in the organic polymer that forms the hydrophilic continuous skeleton is, for example, about 3 mol%, preferably 0.1 to 10 mol%, and more preferably 0.3 to 8 mol%, with respect to all constituent units.

It should be noted that, when the ratio of the crosslinked polymerization residue of divinylbenzene in the organic polymer that forms the hydrophilic continuous skeleton is 0.1 mol% or more, it becomes difficult to decrease the strength of the absorbent A, and, when the ratio of the crosslinked polymerization residue of divinylbenzene is 30 mol% or less, it becomes difficult to decrease the absorption amount of the liquid to be absorbed.

In the absorbent A, the organic polymer that forms the hydrophilic continuous skeleton may be formed of only the constituent unit X and the constituent unit Y, or may be formed of, in addition to the constituent unit X and the constituent unit Y, a constituent unit other than the constituent unit X and the constituent unit Y, such as a polymerization residue of monomers other than (meth)acrylic acid ester and divinylbenzene.

As the constituent unit other than the constituent unit X and the constituent unit Y, for example, there are provided polymerization residues of monomers such as styrene, α-methylstyrene, vinyltoluene, vinylbenzyl chloride, glycidyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, tripropylene glycol di(meth)acrylate, trimethylol propane tri(meth)acrylate and the like.

It should be noted that the ratio of the constituent unit other than the constituent unit X and the constituent unit Y in the organic polymer that forms the hydrophilic continuous skeleton is, for example, 0 to 50 mol%, and preferably 0 to 30 mol%, with respect to all of the constituent units.

Further, it is preferable that the thickness of the hydrophilic continuous skeleton of the absorbent A is 0.1 to 100 µm. When the thickness of the hydrophilic continuous skeleton of the absorbent A is 0.1 µm or more, spaces (pores) for taking in a liquid (bodily fluid) to be absorbed in the porous body is less likely to collapse during absorption, and the absorption amount is less likely to decrease. On the other hand, when the thickness of the hydrophilic continuous skeleton is 100 µm or less, an excellent absorption rate can be easily obtained.

It should be noted that since the pore structure of the hydrophilic continuous skeleton of the absorbent A is a continuous cell structure, the thickness of the continuous skeleton is measured at the thickness evaluation point, which is the cross section of the skeleton appearing in a test piece for electron microscope measurement. The continuous skeleton is formed by spaces between water droplets removed by dehydration and drying after hydrolysis described later, and therefore the skeleton is often polygonal. Therefore, the thickness of the continuous skeleton is defined as the average value of the diameter (µm) of a circle that circumscribes the polygonal cross section. In addition, in rare cases, a small hole may exist in the polygon, but in such cases, the circumscribed circle of the polygonal cross section that surrounds the small hole is measured.

Further, it is preferable that the average diameter of the continuous pores of the absorbent A is 1 to 1000 µm. When the average diameter of the continuous pores of the absorbent A is 1 µm or more, the spaces (pores) for taking in the liquid (bodily fluid) to be absorbed in the porous body become less likely to collapse during absorption, and the absorption rate is less likely to decrease. On the other hand, when the average diameter of the continuous pores is 1000 µm or less, an excellent absorption rate can be easily obtained.

It should be noted that the average diameter (µm) of the continuous pores of the absorbent A can be measured by mercury intrusion porosimetry, and the maximum value of the pore distribution curve obtained by such a mercury intrusion porosimetry is employed. For a sample used for measurement of the average diameter of the continuous pores, regardless of the ionic form of the absorbent A, a sample dried in a vacuum dryer set at a temperature of 50°C for 18 hours or longer is used. It should be noted that the final reaching pressure is set to 0 Torr.A

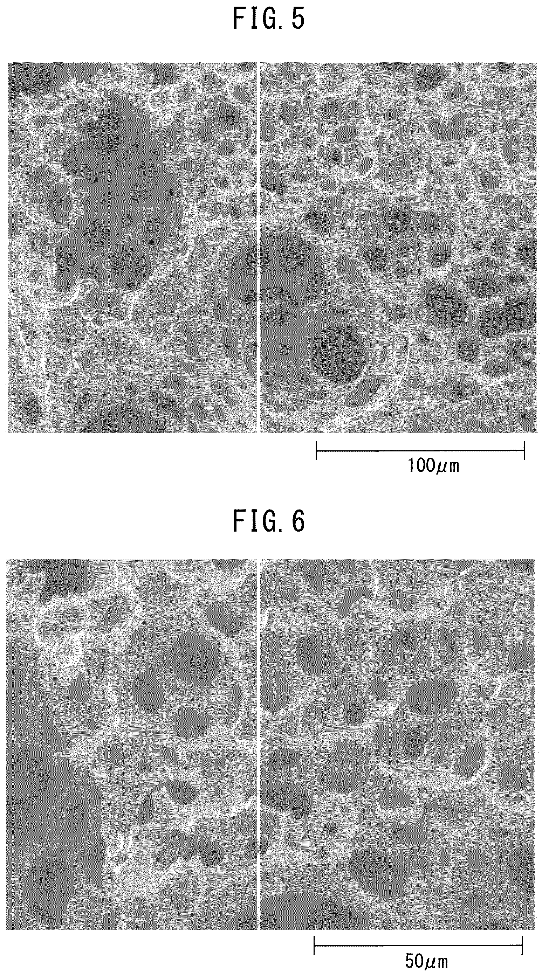

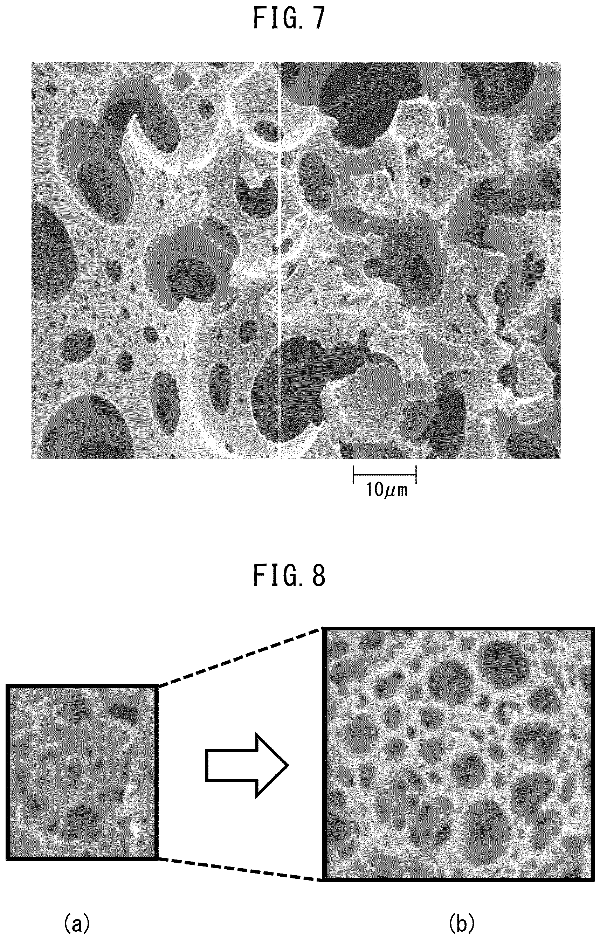

Here, FIG. 3 is an SEM photograph of the absorbent A at a magnification of 50 times, FIG. 4 is an SEM photograph of the absorbent A at a magnification of 100 times, FIG. 5 is an SEM photograph of the absorbent A at a magnification of 500 times, FIG. 6 is an SEM photograph of the absorbent A at a magnification of 1000 times, and FIG. 7 is an SEM photograph of the absorbent A at a magnification of 1500 times.

The absorbent A shown in FIGS. 3 to 7 is an example of an absorbent containing butyl methacrylate as the polymerization monomer and divinylbenzene as the crosslinking monomer, and has a cubic structure having 2 mm sides.

The absorbent A shown in FIGS. 3 to 7 has a large number of bubble-shaped macropores, and further has a portion in which these bubble-shaped macropores overlap each other. The absorbent A has an open cell structure in which portion in which these macropores overlap each other forms a common opening (mesopore), that is, an open cell structure (continuous macropore structure).

The portion in which the macropores overlap each other forms a common opening (mesopore) having an average diameter of 1 to 1000 µm in a dry state, preferably 10 to 200 µm, and particularly preferably 20 to 100 µm, and most of the common openings (mesopores) has an open pore structure.

When the average diameter of the mesopores in the dry state is 1 µm or more, the absorption rate of the liquid to be absorbed becomes more favorable. On the other hand, when the average diameter of the mesopores in the dry state is 1000 µm or less, the absorbent A is less likely to be embrittled.

It should be noted that such an overlap between the macropores is approximately 1 to 12 pieces in one macropore, and is approximately 3 to 10 pieces in many cases.

Further, the absorbent A having such an open cell structure makes it possible to uniformly form a macropore group and a mesopore group, and also has the advantage of making it possible to remarkably increase the pore volume and specific surface area compared with a particle agglomerated porous body such as that described in Japanese Patent Application Publication No. H08-252579.

It should be noted that the total pore volume of the pores of the absorbent A is preferably 0.5 to 50 mL/g, and more preferably 2 to 30 mL/g. When the total pore volume of the absorbent A is 0.5 mL/g or more, spaces (pores) for taking in the liquid (bodily fluid) to be absorbed in the porous body become less likely to collapse during absorption, and the absorption amount and the absorption rate are less likely to decrease. On the other hand, when the total pore volume of the absorbent A is 50 mL/g or less, the strength of the absorbent A is less likely to decrease.

It should be noted that the total pore volume can be measured by the mercury intrusion porosimetry. As a sample for measurement of the total pore volume, regardless of the ionic form of the absorbent A, a sample obtained by drying in a vacuum dryer set at a temperature of 50°C for 18 hours or longer is used. It should be noted that the final reaching pressure is set to 0 Torr.A

Hereinafter, the appearance in a case where the absorbent A and a liquid such as a bodily fluid (hereinafter, simply referred to as a "bodily fluid") come into contact with each other will be described. However, the same applies to a case where the composite absorbent body 4 containing the absorbent A comes into contact with the bodily fluid. Further, since the mass of the absorbed bodily fluid is substantially proportional to the volume of the bodily fluid, in the following description, the mass of the bodily fluid is sometimes simply referred to as the "amount of bodily fluid".

As shown in FIGS. 3 to 7, the continuous pores of the absorbent A are pores in which a plurality of pores are continuous with each other, and the fact that a large number of pores are located can be visually recognized from the external appearance as well. When the bodily fluid such as urine or menstrual blood comes into contact with the absorbent A having a large number of pores, the hydrophilic continuous skeleton first instantaneously takes in a part of the bodily fluid by osmotic pressure and extends (that is, expands). The continuous skeleton extends in substantially all directions.

Here, FIG. 8 is a cross-sectional photograph taken with a scanning electron microscope (SEM) showing the appearance of the absorbent A before and after water absorption. In FIG. 8, the SEM photograph (a) on the left side is the absorbent A before water absorption, and the SEM photograph (b) on the right side is the absorbent A after water absorption.

As shown in FIG. 8, in the absorbent A, as the outer shape of the absorbent A increases due to the extension of the continuous skeleton during water absorption, the size of the pores also increases. When the size of the pores is increased in this manner, the volume in the pore is increased, and therefore, the amount of bodily fluid that can be stored in the pores is also increased. That is, the absorbent A which is increased due to the absorption of a predetermined amount of bodily fluid in this manner is capable of further absorbing a predetermined amount of bodily fluid into the expanded pores due to the capillary phenomenon.

The absorbent A exhibits a unique water absorption behavior that, when water (bodily fluid) is absorbed, the water is taken into the hydrophilic continuous skeleton and then taken into the continuous pores to be absorbed.

It should be noted that, while the bodily fluid absorbed into the hydrophilic continuous skeleton of the absorbent A is less likely to be released from the continuous skeleton (that is, less likely to be separated), the bodily fluid absorbed into the continuous pores is likely to be separated. Therefore, in the composite absorbent body, the bodily fluid absorbed into the continuous pores is separated, transferred to a superabsorbent polymer (SAP) having a high water retention capacity, and steadily held in the SAP.

Here, regarding the amount of bodily fluid absorbed into the continuous skeleton of the absorbent A and the amount of bodily fluid absorbed into the continuous pores, in the total amount of bodily fluid absorbed by the absorbent A, the amount of bodily fluid separated from the absorbent A (the amount of separation) in a centrifugation treatment (at 150 G for 90 seconds) becomes the amount of bodily fluid absorbed into the continuous pores, and the amount of bodily fluid excluding the amount of bodily fluid (that is, the amount of bodily fluid not separated from the absorbent A in the centrifugation treatment) becomes the amount of bodily fluid absorbed into the continuous skeleton.

Further, in the bodily fluid absorbed by the absorbent A, the amount of bodily fluid that remains in the pores is larger than the amount of bodily fluid absorbed in the hydrophilic continuous skeleton. Since most of the bodily fluid absorbed in the absorbent A is absorbed by storing the bodily fluid in the pores due to the capillary phenomenon, the higher the porosity (the volume of the pores with respect to the volume of the absorbent A), which is the ratio of the void volume of the pores (total pore volume), the more the bodily fluid can be absorbed. It should be noted that it is preferable that the porosity is 85% or more.

For example, when the porosity of the absorbent A shown in FIGS. 3 to 7 described above is calculated, the following is obtained.

First, the specific surface area of the absorbent A obtained by the mercury intrusion porosimetry is 400 m²/g, and the pore volume is 15.5 mL/g. This pore volume of 15.5 mL/g means that the volume of the pores in 1 g of the absorbent A is 15.5 mL.

Here, in a case where the specific gravity of the absorbent A is 1 g/mL, the volume of the pores occupied in 1 g of the absorbent A, that is, the pore volume, is 15.5 mL, and the volume of 1 g of the absorbent A is 1 mL.

Then, the total volume (volume) of 1 g of the absorbent A becomes 15.5 + 1 (mL), and the ratio of the pore volume in the total volume becomes the porosity. Therefore, the porosity of the absorbent A becomes 15.5/(15.5 + 1) x 100 ≤ 94%.

The absorbent A having such a hydrophilic continuous skeleton and continuous pores, that is, the polymer absorbent is applied to a composite absorbent body for absorbing a bodily fluid such as urine or menstrual blood, such as the composite absorbent body 4 of the light incontinence pad 1 described above, in the form of, for example, a particle, a sheet, or the like.

Further, as described above, this polymer absorbent exhibits a unique water absorption behavior that when water is absorbed, the water is taken into the hydrophilic continuous skeleton and then taken into the continuous pores. Therefore, a large amount of bodily fluid can be instantaneously absorbed, and further the absorbed bodily fluid (mainly the bodily fluid absorbed in the continuous pores) can be transferred to the SAP having a high water retention capacity and can be steadily held in the SAP. Therefore, the composite absorbent body to which such a polymer absorbent is applied can exhibit high absorption performance as an absorbent body.

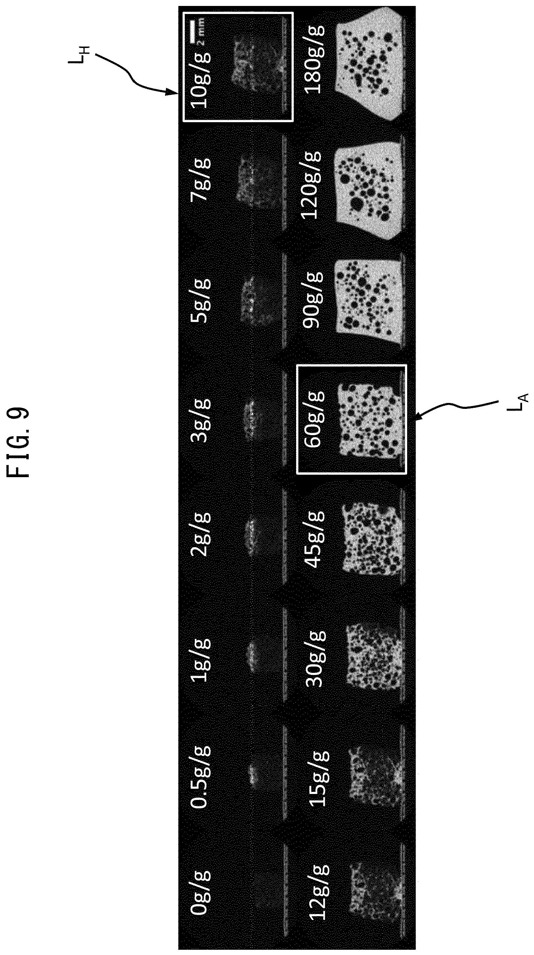

Here, the polymer absorbent as an example of the present invention, two types of superabsorbent polymers (SAP (A) and SAP (B)) as comparative examples, and an Infinity particle body as a comparative example are prepared, and the absorption state when a predetermined amount of distilled water is dropped to each of these materials is observed by photographing using a 3D micro X-ray CT apparatus (CosmoScan FX (manufactured by Rigak Corporation)). At the time of photographing, a sample of the polymer absorbent or the like is fixed to the bed of the apparatus with a double-sided tape, and distilled water is added from above. The photographing conditions are as follows: tube voltage: 90 kV, tube current: 88 µA, irradiation time: 2 minutes, resolution: 20 µm, matrix: 512 × 512 × 512. The observation results are shown in FIGS. 9 to 11.

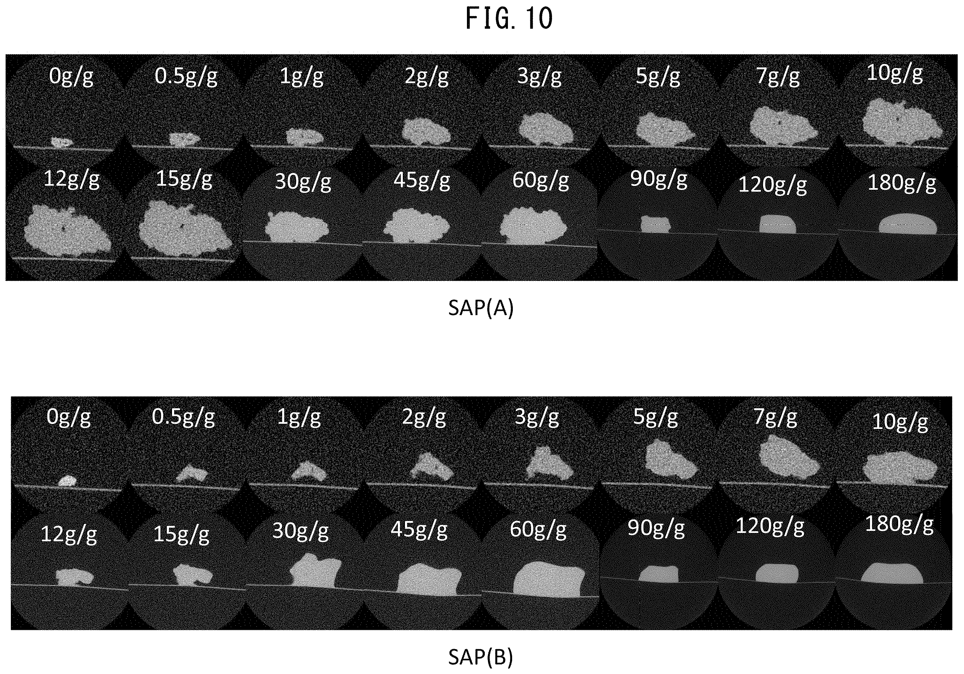

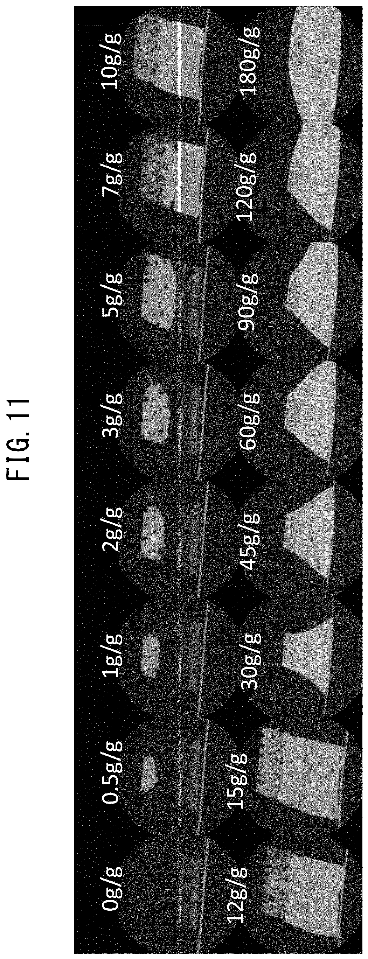

It should be noted that FIG. 9 is a CT image showing the absorption state of the absorbent A in each dropping amount of distilled water, FIG. 10 is a CT image showing the absorption states of two types of SAPs in each dropping amount of distilled water, and FIG. 11 is a CT image showing the absorption state of the Infinity particle body in each dropping amount of distilled water. In FIGS. 9 to 11, the white portion of each CT image corresponds to a portion in which water is absorbed, and the amount of each dropped distilled water is represented by the water absorption amount (mass) per unit mass of the absorbent such as a polymer absorbent, that is, the water absorption ratio (g/g).

It should be noted that the above Infinity particle body is an absorbent manufactured by P&G Corporation and has a structure similar to the polymer absorbent (foamed structure), but, unlike the polymer absorbent, the Infinity particle body does not have a function of absorbing water and expanding.

As shown in FIG. 9, in the absorbent A of the example of the present invention, as the dropping amount (water absorption ratio) of distilled water increases, water absorption proceeds in the hydrophilic continuous skeleton, and the continuous skeleton extends, so that the outer shape of the absorbent A becomes large. Then, after the water absorption ratio reaches 10 g/g (that is, 25% water absorption), which is a water retention limit L_{H} of the absorbent A, water absorption proceeds in the continuous pores, and a larger amount of water can be absorbed. It should be noted that, after the water absorption ratio reaches 60 g/g, which is a water absorption limit L_{A} of the absorbent A, as shown in FIG. 9, water is less likely to be absorbed and at a water absorption capacity of 90 g/g, water overflows.

On the other hand, in the SAPs of the comparative examples, as shown in FIG. 10, all of the two types of SAPs absorb water while uniformly diffusing the water into the SAPs as the dropping amount (water absorption ratio) of distilled water increases, and therefore, the stepwise water absorption behavior like that of the absorbent A of the example of the present example is not shown. Further, in the Infinity particle body of the comparative example, as shown in FIG. 11, as the dropping amount of distilled water increases, water absorption proceeds from the upper part side of the Infinity particle body, and this Infinity particle body also does not exhibit the stepwise water absorption behavior like that of the absorbent A of the example of the present invention.

As described above, the absorbent A of the example of the present invention exhibits the above unique water absorption behavior that is not present in conventional SAP or Infinity particle bodies, and it can be found that a large amount of bodily fluid can be instantaneously absorbed and further excellent absorption performance that the absorbed bodily fluid can be transferred to the SAP having high water retention capacity can be exhibited.

It should be noted that, in the present invention, it is preferable that a volume of the expanded polymer absorbent is larger than a volume of the water taken in at the time of water absorption of the polymer absorbent at the water retention limit L_{H}. Since such a polymer absorbent can take a larger amount of water into the continuous pores, the composite absorbent body including the polymer absorbent can exhibit higher absorption performance as an absorbent body. It should be noted that the properties of such a polymer absorbent can be embodied by appropriately adjusting, for example, the total pore volume of the above-described continuous pores, the porosity of the polymer absorbent, and the like.

Here, the volume of the expanded polymer absorbent at the time of water absorption at the water retention limit L_{H} and the volume of the water taken into the polymer absorbent at the time of water absorption at the water retention limit L_{H} can be measured as follows.

### <Method for Measuring Volume of Expanded Polymer Absorbent at Water Absorption at Water Retention Limit LH>

(1) 1 g of a sample (polymer absorbent) for measurement is sealed in a 10 cm square mesh bag (N-No.255HD 115, manufactured by NBC Mesh Tech Co., Ltd.). It should be noted that the mass (g) of the mesh bag is measured in advance.
(2) The mesh bag containing the sample is immersed in physiological saline (0.9% sodium chloride aqueous solution) for 1 hour.
(3)The mass (g) after the mesh bag is suspended for 5 minutes and drained is measured.
(4) After draining, the mesh bag is subjected to a centrifugation treatment at 150 G for 90 seconds, and the mass (g) of the mesh bag after the centrifugation treatment is measured.
(5) The water retention amount (g) of the sample is calculated by subtracting the mass of the sample (= 1 g) and the total mass of the mesh bag from the mass of the mesh bag after the centrifugation treatment, and the water retention amount is further divided by the mass of the sample (= 1 g) to obtain the water retention amount (g/g) per unit mass of the sample (polymer absorbent). The water retention amount per unit mass is defined as a water retention limit L_{H} (g/g).
(6) Separately, 30 particles of the sample (polymer absorbent) for measurement are taken out to measure the mass thereof, and further the mass is divided by the number of particles (30 particles) to calculate the average mass (g) per particle of the sample.
(7) One particle that best matches the value of the average mass is selected from the 30 particles of the sample, and the one particle of the sample is fixed to the bed of a 3D micro X-ray CT apparatus (CosmoScan FX, manufactured by Rigaku Corporation) with a double-sided tape.
(8) The quantity (g) of distilled water corresponding to the water retention limit L_{H} obtained in (5) above is added to the fixed one particle of the sample from above, and the distilled water is incorporated into the one particle of the sample.
(9) One particle of the sample containing the distilled water is observed with the 3D microx X-ray CT apparatus (tube voltage: 90 kV, tube current: 88 µA, irradiation time: 2 minutes, resolution: 20 µm, matrix: 512 × 512 × 512), and the volume (cm³) of the one particle of the expanded sample at the water retention limit L_{H} is calculated from the obtained CT image.

It should be noted that, as the volume of the water taken into the polymer absorbent at the time of water absorption at the water retention limit L_{H}, the volume (cm³) of the amount (g) of the water retention limit L_{H} is calculated, assuming that 1 g of distilled water is 1 cm³.

Further, in a case where the sample for measurement (polymer absorbent) is collected from a product of a sanitary product and used, the sample for measurement (polymer absorbent) can be obtained according to the following <Method for Collecting Sample for Measurement (Polymer Absorbent)>.

### <Method for Collecting Sample for Measurement (Polymer Absorbent)>

(1) The top sheet or the like is peeled off from the product of the sanitary product to expose the absorbent body.
(2) The object to be measured (polymer absorbent) is dropped from the exposed absorbent body, and a substance other than the object to be measured (in a particulate state) (for example, pulp, synthetic resin fibers, or the like) is removed with tweezers or the like.
(3) A microscope or a simple loupe is used as magnifying observation means, and the object to be measured is collected using the tweezers or the like, while observing at a magnification capable of recognizing the difference from SAP or at a magnification capable of visually recognizing the pores of the porous body. It should be noted that the magnification of the simple loupe is not particularly limited as long as the pores of the porous body can be visually recognized, and, for example, a magnification of 25 to 50 times can be given.
(4) The object to be measured collected in this manner is used as the sample for measurement in various measurement methods.

Further, it is preferable that water absorption amount of the continuous pores is larger than the water absorption amount of the continuous skeleton in the polymer absorbent. Since such a polymer absorbent can take a larger amount of water into the continuous pores, the composite absorbent body containing the polymer absorbent can exhibit higher absorption performance as an absorbent body. It should be noted that the properties of such a polymer absorbent can be embodied by appropriately adjusting, for example, the total pore volume of the above-described continuous pores, the porosity of the polymer absorbent, and the like.

Here, the water absorption amount of the continuous pores and the water absorption amount of the continuous skeleton of the polymer absorbent can be measured as follows.

### <Method for Measuring Water Absorption Amount of Continuous Pores and Water Absorption Amount of Continuous Skeleton of Polymer Absorbent>

(1) 1 g of a sample (polymer absorbent) for measurement is sealed in a 10 cm square mesh bag (N-No.255HD 115, manufactured by NBC Mesh Tech Co., Ltd.). It should be noted that the mass (g) of the mesh bag is measured in advance.
(2) The mesh bag containing the sample is immersed in physiological saline (0.9% sodium chloride aqueous solution) for 1 hour.
(3)The mass (g) after the mesh bag is suspended for 5 minutes and drained is measured.
(4) The water absorption amount (g) of the sample is calculated by subtracting the mass (= 1 g) of the sample and the total mass of the mesh bag from the mass of the mesh bag after draining measured in (3) above, and the water absorption amount is further divided by the mass (= 1 g) of the sample to obtain the water absorption amount (g/g) per unit mass of the sample (polymer absorbent). The water absorption amount per unit mass is defined as a water absorption limit L_{A} (g/g).
(5) Further, the mesh bag after draining in (3) above is subjected to a centrifugation treatment at 150 G for 90 seconds, and the mass (g) of the mesh bag after the centrifugation treatment is measured.
(6) The water retention amount (g) of the sample is calculated by subtracting the mass (= 1 g) of the sample and the total mass of the mesh bag from the mass of the mesh bag after the centrifugation treatment measured in (5) above, and the water retention amount is further divided by the mass (= 1 g) of the sample to obtain the water retention amount (g/g) per unit mass of the sample (polymer absorbent). This water retention amount per unit mass becomes the water retention limit L_{H} (g/g), and this water retention limit L_{H} is defined as the "water absorption amount of the continuous skeleton".
(7) Then, the amount (g/g) obtained by subtracting the water retention limit L_{H} from the water absorption limit L_{A} obtained in (4) above is defined as the "water absorption amount of the continuous pores".

It should be noted that it is preferable that the mass ratio of the water absorption amount of the continuous pores and the water absorption amount of the continuous skeleton in the polymer absorbent is 60:40 to 95:5. When the mass ratio of the water absorption amount of the continuous pores and the water absorption amount of the continuous skeleton in the polymer absorbent is within such a specific range, water can be more steadily taken into the continuous skeleton and then taken into the continuous pores when the polymer absorbent absorbs the water. It should be noted that the properties of such a polymer absorbent can also be embodied by appropriately adjusting, for example, the total pore volume of the continuous pores, the porosity of the polymer absorbent, and the like.

Further, it is preferable that the polymer absorbent has a relatively high absorption rate from immediately after the start of water absorption until reaching the water retention limit, and a relatively low absorption rate after the water retention limit.

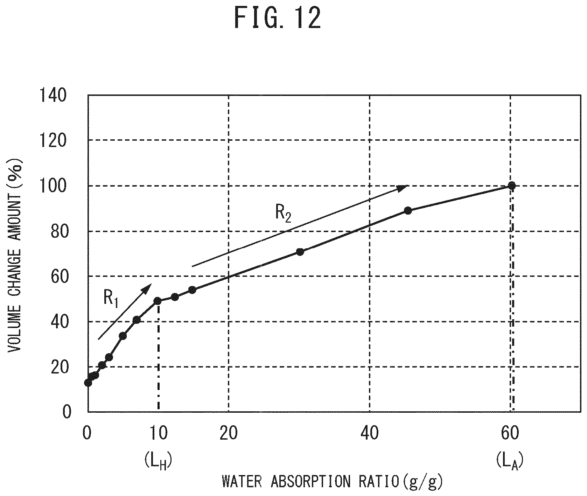

Here, FIG. 12 is a graph showing the relationship between the water absorption ratio and the volume change amount of the absorbent A which is an example of the polymer absorbent of the present invention.

As shown in FIG. 12, it can be found that while the volume change amount of the absorbent A rapidly increases as the water absorption ratio (that is, the absorption rate R₁ of water is high) increases after the start of water absorption, the volume change amount gradually increases (that is, the absorption rate R₂ of water is low) after the water absorption capacity reaches 10 g/g, which is the water retention limit L_{H} of the absorbent A.

As described above, while in the initial stage of water absorption from immediately after the start of water absorption until reaching the water retention limit L_{H}, the absorption rate of the absorbent A is relatively high, and the bodily fluid can be instantaneously taken into the continuous skeleton, in the latter stage of water absorption after the water retention limit L_{H}, the absorption rate of the polymer absorbent is relatively low and the bodily fluid can be steadily taken into the continuous pores expanded due to the expansion of the continuous skeleton. Accordingly, the absorbent A can more reliably exhibit high absorption performance as an absorbent body.

It should be noted that, the volume change amount of the polymer absorbent can be obtained by measuring the volume of the expanded polymer absorbent at each water absorption ratio according to the above-described <Method for Measuring Volume of Expanded Polymer Absorbent at Water Absorption at Water Retention Limit L_{H}>, dividing the volume of the expanded polymer absorbent by the volume of the polymer absorbent before water absorption, and multiplying the volume by 100.

Hereinafter, a method for manufacturing such a polymer absorbent will be described in detail using the above-described absorbent A as an example.

### [Method for Manufacturing Polymer Absorbent]

As shown in FIG. 2, the above-described absorbent A can be obtained by allowing the absorbent A to pass through a crosslinking polymerization step and a hydrolysis step. Hereinafter, each of these steps will be described.

### (Crosslinking Polymerization Step)

First, an oil-soluble monomer for crosslinking polymerization, a crosslinking monomer, a surfactant, water, and optionally a polymerization initiator are mixed to obtain a water-in-oil emulsion. This water-in-oil emulsion is an emulsion in which water droplets are dispersed in an oil phase that serves as a continuous phase.

As shown in the upper figure of FIG. 2, in the above-described absorbent A, a monolith A is obtained by performing crosslinking polymerization using butyl methacrylate, which is (meth)acrylic acid ester, as an oil-soluble monomer, divinylbenzene as a crosslinking monomer, sorbitan monooleate as a surfactant, and further isobutyronitrile as a polymerization initiator.

Specifically, in the absorbent A, as shown in the upper figure of FIG.2, first, 9.2 g of tert-butyl methacrylate as an oil-soluble monomer, 0.28 g of divinylbenzene as a crosslinking monomer, 1.0 g of sorbitan monooleate (hereinafter, abbreviated as "SMO") as a surfactant, and 0.4 g of 2,2'-azobis(isobutyronitrile) as a polymerization initiator are mixed and uniformly dissolved.

Next, the mixture of t-butyl methacrylate, divinylbenzene, SMO, and 2,2'-azobis(isobutyronitrile) is added to 180 g of pure water, and a vacuum stirring defoaming mixer (manufactured by EME. Corp.), which is a planetary motion type stirring device, is used to perform stirring under reduced pressure to obtain a water-in-oil emulsion.

Further, the emulsion is rapidly transferred to a reaction vessel, sealed, and left to stand still and polymerized at 60°C for 24 hours. After completion of the polymerization, the content is taken out, extracted with methanol, and dried under reduced pressure to obtain a monolith A having a continuous macropore structure. It should be noted that, as a result of observing the internal structure of the monolith A by SEM, the monolith A had an open cell structure and the thickness of the continuous skeleton is 5.4 µm. Further, the average diameter of the continuous pores measured by the mercury intrusion porosimetry is 36.2 µm, and the total pore volume is 15.5 mL/g.

It should be noted that the content of divinylbenzene with respect to all the monomers is preferably 0.3 to 10 mol%, and more preferably 0.3 to 5 mol%. Further, the ratio of divinylbenzene to the total of butyl methacrylate and divinylbenzene is preferably 0.1 to 10 mol%, and more preferably 0.3 to 8 mol%. It should be noted that in the above-described absorbent A, the ratio of butyl methacrylate with respect to the total of butyl methacrylate and divinylbenzene is 97.0 mol% and the ratio of divinylbenzene with respect to the total of butyl methacrylate and divinylbenzene is 3.0 mol%.

The amount of the surfactant added can be set depending on the type of the oil-soluble monomer and the size of the desired emulsion particle (macropore), and it is preferable that the amount of the surfactant is set to be in a range of about 2 to 70% with respect to the total amount of the oil-soluble monomer and the surfactant.

It should be noted that, in order to control the bubble shape and the size of the monolith A, alcohols such as methanol and stearyl alcohol; carboxylic acids such as stearic acid; hydrocarbons such as octane, dodecane, and toluene; cyclic ethers such as tetrahydrofuran and dioxane, and the like may coexist in the polymerization system.

Further, the mixing method for forming the water-in-oil emulsion is not particularly limited, and for example, any mixing method such as a method of mixing each component all together at once, and a method in which oil-soluble components, which are an oil-soluble monomer, a surfactant, and an oil-soluble polymerization initiator, and water-soluble components, which are water or a water-soluble polymerization initiator, are uniformly dissolved separately, and then oil-soluble components and water-soluble components are mixed can be adopted.

Further, the mixing device for forming the emulsion is not particularly limited, and any device such as an ordinary mixer, homogenizer, or high pressure homogenizer can be used according to the desired emulsion particle diameter. Further, a so-called planetary type stirring device in which an object to be treated is placed in a mixing container, and the object to be treated is stirred and mixed by rotating the mixing container in a tilted state while revolving around the revolution axis can be used.

Further, the mixing conditions are not particularly limited, and any stirring rotation rate, any stirring time, and the like can be set depending on the desired emulsion particle diameter. It should be noted that, in the above-described planetary type stirring device, water droplets can be uniformly generated in the W/O emulsion, and any average diameter can be set within a wide range.

As the polymerization conditions of the water-in-oil emulsion, various conditions can be adopted depending on the type of monomer or initiator and the like. For example, in a case of using azobisisobutyronitrile, benzoyl peroxide, potassium persulfate, or the like as the polymerization initiator, polymerization may be conducted by heating at a temperature of 30°C to 100°C for 1 to 48 hours in a sealed container under an inert atmosphere. In a case of using hydrogen peroxide-ferrous chloride, sodium persulfate-acid sulfite, or the like as the polymerization initiator, polymerization may be conducted at a temperature of 0 to 30°C for 1 to 48 hours in a sealed container under an inert atmosphere.

It should be noted that, after the polymerization, the content is taken out and subjected to Soxhlet extraction with a solvent such as isopropanol to remove the unreacted monomer and the residual surfactant, and thus the monolith A shown in the middle figure of FIG. 2 is obtained.

### (Hydrolysis Step)

Subsequently, a step (hydrolysis step) of hydrolyzing the monolith A (crosslinked polymer) to obtain the absorbent A will be described.

First, the monolith A is immersed in dichloroethane to which zinc bromide is added, stirred at 40°C for 24 hours, then brought into contact with methanol, 4% hydrochloric acid, a 4% aqueous solution of sodium hydroxide, and water in this order for hydrolysis, and then dried. Thus, a block-shaped absorbent A is obtained. Further, the block-shaped absorbent A is pulverized to a predetermined size to obtain a particulate absorbent A. It should be noted that the form of the absorbent A is not limited to a particulate form, and, for example, the absorbent A may be shaped into a sheet shape while or after drying.

Further, the method for hydrolyzing the monolith A is not particularly limited, and various methods can be adopted. For example, there is a method of bringing a solvent into contact with a strong base such as sodium hydroxide, or a method of bringing a solvent into contact with a hydrohalic acid such as hydrochloric acid, a Brönsted acid such as sulfuric acid, nitric acid, trifluoroacetic acid, methanesulfonic acid, or p-toluenesulfonic acid), or a Lewis acid such as zinc bromide, aluminum chloride, aluminum bromide, titanium (IV) chloride, cerium chloride/sodium iodide, or magnesium iodide) using any of various solvent including aromatic solvents such as toluene and xylene, halogen-based solvents such as chloroform and dichloroethane, ether-based solvents such as tetrahydrofuran and isopropyl ether, amide-based solvents such as dimethylformamide and dimethylacetamide, alcohol-based solvents such as methanol and ethanol, carboxylic acid-based solvents such as acetic acid and propionic acid, or water as the solvent.

Further, among the polymerization raw materials of the organic polymer that forms the hydrophilic continuous skeleton of the absorbent A, the (meth)acrylic acid ester is not particularly limited, C1 to C10 (that is, containing 1 to 10 carbon atoms) alkyl ester of (meth)acrylic acid is preferable, and C4 (that is, containing 4 carbon atoms) alkyl ester of (meth)acrylic acid is particularly preferable.

It should be noted that as a C4 alkyl ester of (meth)acrylic acid, there are provided (meth)acrylic acid t-butyl ester, (meth)acrylic acid n-butyl ester, and (meth)acrylic acid iso-butyl ester.

Further, the monomer used for the crosslinking polymerization may be only (meth)acrylic acid ester and divinylbenzene, or may include (meth)acrylic acid ester and divinylbenzene, and additionally, a monomer other than (meth)acrylic acid ester and divinylbenzene.

In the latter case, although the monomer other than (meth)acrylic acid ester and divinylbenzene styrene is not particularly limited, for example, there are provided α-methylstyrene, vinyltoluene, vinylbenzyl chloride, glycidyl (meth)acrylate, 2-ethylhexyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, trimethlol propane tri(meth)acrylate, and the like.

It should be noted that, the ratio of the monomer other than (meth)acrylic acid ester and divinylbenzene in all monomers used for the crosslinking polymerization is preferably 0 to 80 mol%, and more preferably 0 to 50 mol%.

Further, the surfactant is not limited to the above-described sorbitan monooleate, and any surfactant that can form a water-in-oil (W/O) emulsion when a monomer for crosslinking polymerization and water are mixed can be used. As such a surfactant, for example, there are provided nonionic surfactants such as sorbitan monolaurate, sorbitan monopalmitate, sorbitan monostearate, sorbitan trioleate, polyoxyethylene group nonylphenyl ether, polyoxyethylene group stearyl ether, and polyoxyethylene group sorbitan monooleate, anionic surfactants such as potassium oleate, sodium dodecylbenzene sulfonate, and dioctyl sodium sulfosuccinate, cationic surfactants such as distearyl dimethyl ammonium chloride, and amphoretic surfactants such as lauryl dimethyl betaine. These surfactants may be used alone or in combination of two or more types.

In addition, a compound that generates radicals by heat and light irradiation is suitably used as the polymerization initiator. The polymerization initiator may be water-soluble or oil-soluble. For example, there are provided azobis (4-methoxy-2,4-dimethylvaleronitrile), azobisisobutyronitrile, azobisdimethylvaleronitrile, azobiscyclohexanenitrile, azobiscyclohexanecarbonitrile, azobis (2-methylpropionamidine) dihydrochloride, benzoyl peroxide, potassium persulfate, ammonium persulfate, hydrogen peroxide-ferrous chloride, sodium persulfate-sodium acid sulfite, and tetramethylthiuram disulfide. However, in some cases, there is a system in which polymerization proceeds with only heating or only light irradiation even when the polymerization initiator is not added. Therefore, in such a system, the addition of the polymerization initiator is not necessary.

It should be noted that, in addition to the light incontinence pad of the above-described embodiment, the composite absorbent body of the present invention can be applied to various sanitary products such as underpants-shaped disposable diapers, tape-type disposable diapers, sanitary napkins, absorbent panty liners, absorbent pads (for example, bedsore pads, puerperal pads and the like), absorbent sheets, breast pads, disposable diapers for pets, absorbent pads for pets, excrement disposal sheets for pets, wet sheets, wet tissues, cosmetic wiping sheets, masks, and the like. Therefore, the bodily fluid that is the liquid to be absorbed of the composite absorbent body is a liquid discharged from a wearer of a sanitary product, and for example, there are provided urine, sweat, feces, menstrual blood, vaginal discharge, breast milk, blood, exudate, and the like.

In addition, the present invention is not limited to the above-described embodiments and the like, and appropriate combinations, substitutions, modifications, and the like can be made without departing from the aspect, gist, and the like of the present invention.

### REFERENCE SIGNS LIST

1: Light incontinence pad
2: top sheet
3: back sheet
4: composite absorbent body

## Claims

1. A composite absorbent body (4) for absorbing a bodily fluid, comprising:
a polymer absorbent having a hydrophilic continuous skeleton and a continuous pore; and
a superabsorbent polymer;
wherein when the polymer absorbent absorbs water, the water is taken into the continuous pore after the water is taken into the continuous skeleton;
wherein the polymer absorbent is a hydrolysate of a crosslinked polymer of a (meth)acrylic acid ester and divinylbenzene which is a compound containing two or more vinyl groups in one molecule; and
wherein a ratio of a crosslinked polymerization residue of the divinylbenzene in an organic polymer that forms the hydrophilic continuous skeleton is 0.1 to 30 mol % with respect to all the constituent units.

2. The composite absorbent body according to Claim 1, wherein
a volume of an expanded polymer absorbent is larger than a volume of a water taken in at a time of water absorption of the polymer absorbent at a water retention limit.

3. The composite absorbent body according to Claim 1 or 2, wherein
a water absorption amount of the continuous pore is larger than a water absorption amount of the continuous skeleton in the polymer absorbent.

4. The composite absorbent body according to any one of Claims 1 to 3, wherein
a mass ratio between a water absorption amount of the continuous pore and a water absorption amount of the continuous skeleton in the polymer absorbent is 60:40 to 95:5.

5. The composite absorbent body according to any one of Claims 1 to 4, wherein
the polymer absorbent has a higher absorption rate from immediately after start of water absorption until reaching a water retention limit, compared to the absorption rate after the water retention limit has been reached.

6. The composite absorbent body according to any one of Claims 1 to 5, wherein
the polymer absorbent is a monolithic absorbent.

7. A sanitary product (1) comprising:
the composite absorbent body (4) according to any one of Claims 1 to 6.

## Patentansprüche

1. Zusammengesetzter Saugkörper (4) zur Absorption einer Körperflüssigkeit, der Folgendes umfasst:
ein Polymerabsorptionsmittel, das ein hydrophiles kontinuierliches Gerüst und eine kontinuierliche Pore aufweist; und
ein superabsorbierendes Polymer;
wobei, wenn das Polymerabsorptionsmittel Wasser absorbiert, das Wasser in die kontinuierliche Pore aufgenommen wird, nachdem das Wasser in das kontinuierliche Gerüst aufgenommen wird;
wobei das Polymerabsorptionsmittel ein Hydrolysat eines vernetzten Polymers eines (Meth)acrylsäureesters und Divinylbenzols, das eine Verbindung ist, die zwei oder mehr Vinylgruppen in einem Molekül enthält, ist; und
wobei ein Verhältnis eines vernetzten Polymerisationsrestes des Divinylbenzols in einem organischen Polymer, das das hydrophile kontinuierliche Gerüst bildet, 0,1 bis 30 mol-% in Bezug auf alle Bestandteileinheiten beträgt.

2. Zusammengesetzter Saugkörper nach Anspruch 1, wobei
ein Volumen eines ausgeweiteten Polymerabsorptionsmittels größer ist als ein Volumen eines Wassers, das zu einem Zeitpunkt von Wasserabsorption des Polymerabsorptionsmittels bei einer Wasserretentionsgrenze aufgenommen wird.

3. Zusammengesetzter Saugkörper nach Anspruch 1 oder 2, wobei
eine Wasserabsorptionsmenge der kontinuierlichen Pore größer ist als eine Wasserabsorptionsmenge des kontinuierlichen Gerüsts in dem Polymerabsorptionsmittel.

4. Zusammengesetzter Saugkörper nach einem der Ansprüche 1 bis 3, wobei
ein Massenverhältnis zwischen einer Wasserabsorptionsmenge der kontinuierlichen Pore und einer Wasserabsorptionsmenge des kontinuierlichen Gerüsts in dem Polymerabsorptionsmittel 60 : 40 bis 95 : 5 beträgt.

5. Zusammengesetzter Saugkörper nach einem der Ansprüche 1 bis 4, wobei
das Polymerabsorptionsmittel eine höhere Absorptionsrate von unmittelbar nach Beginn von Wasserabsorption bis Erreichen einer Wasserretentionsgrenze aufweist im Vergleich zu der Absorptionsrate, nachdem die Wasserretentionsgrenze erreicht wurde.

6. Zusammengesetzter Saugkörper nach einem der Ansprüche 1 bis 5, wobei
das Polymerabsorptionsmittel ein monolithisches Absorptionsmittel ist.

7. Hygieneprodukt (1), das Folgendes umfasst:
den zusammengesetzten Saugkörper (4) nach einem der Ansprüche 1 bis 6.

## Revendications

1. Corps absorbant composite (4) destiné à absorber un fluide corporel, comportant :
un absorbant polymère ayant un squelette continu hydrophile et un pore continu ; et
un polymère superabsorbant ;
dans lequel, quand l'absorbant polymère absorbe de l'eau, l'eau est prise dans le pore continu après que l'eau a été prise dans le squelette continu ;
dans lequel l'absorbant polymère est un hydrolysat d'un polymère réticulé d'un ester d'acide (méth)acrylique et de divinylbenzène qui est un composé contenant deux ou plusieurs groupes vinyle dans une molécule ; et
dans lequel un rapport d'un résidu de polymérisation réticulé du divinylbenzène dans un polymère organique qui forme le squelette continu hydrophile va de 0,1 à 30 % en moles par rapport à toutes les unités constituantes.

2. Corps absorbant composite selon la revendication 1, dans lequel
un volume d'un absorbant polymère expansé est supérieur à un volume d'eau prise au moment de l'absorption d'eau de l'absorbant polymère à une limite de rétention d'eau.

3. Corps absorbant composite selon la revendication 1 ou la revendication 2, dans lequel
une quantité d'absorption d'eau du pore continu est supérieure à une quantité d'absorption d'eau du squelette continu dans l'absorbant polymère.

4. Corps absorbant composite selon l'une quelconque des revendications 1 à 3, dans lequel
un rapport massique entre une quantité d'absorption d'eau du pore continu et une quantité d'absorption d'eau du squelette continu dans l'absorbant polymère est compris entre 60:40 et 95:5.

5. Corps absorbant composite selon l'une quelconque des revendications 1 à 4, dans lequel
l'absorbant polymère présente un taux d'absorption plus élevé à partir du moment immédiatement après le début de l'absorption d'eau jusqu'au moment où une limite de rétention d'eau est atteinte, comparé au taux d'absorption après que la limite de rétention d'eau a été atteinte.

6. Corps absorbant composite selon l'une quelconque des revendications 1 à 5, dans lequel
l'absorbant polymère est un absorbant monolithique.

7. Produit hygiénique (1) comportant :
le corps absorbant composite (4) selon l'une quelconque des revendications 1 à 6.
